# EUROPÄISCHE PATENTSCHRIFT

(11) **EP 3 049 125 B1**
(45) Veröffentlichungstag und Bekanntmachung des Hinweises auf die Patenterteilung: **27.02.2019**
(21) Anmeldenummer: 14772150.0
(22) Anmeldetag: 24.09.2014
(51) Int. Cl.: A61M 1/28

(54) **SENSORIK ZUR DETEKTION VON PHASEN UND/ODER PHASENÜBERGÄNGEN BEI PERITONEALDIALYSEBEHANDLUNGEN**
SENSOR SYSTEM FOR DETECTING PHASES AND/OR PHASE TRANSITIONS IN PERITONEAL DIALYSIS
ENSEMBLE DE CAPTEURS DE DÉTECTION DE PHASES ET/OU DE TRANSITIONS DE PHASES LORS DE TRAITEMENTS PAR DIALYSE PÉRITONÉALE

(30) Priorität: 28.09.2013 DE 102013016204
(43) Veröffentlichungstag der Anmeldung: 03.08.2016
(73) Patentinhaber: Fresenius Medical Care Deutschland GmbH, 61352 Bad Homburg (DE)
(72) Erfinder: GRÖBER, Tobias, 63150 Heusenstamm (DE); WABEL, Peter, 64287 Darmstadt (DE); WIESKOTTEN, Sebastian, 64560 Riedstadt (DE)
(74) Vertreter: Breuninger, Marcus
(86) Internationale Anmeldenummer: PCT/EP2014/070342
(87) Internationale Veröffentlichungsnummer: WO 2015/044185

(56) Entgegenhaltungen:
- WO-A1-96/37243
- WO-A2-2014/109900
- CA-A1- 2 223 846

## Beschreibung

Die Erfindung richtet sich auf eine Sensorik eines medizinischen Systems, insbesondere eines gravimetrisch arbeitenden und manuell bedienbaren Systems, für die kontinuierliche ambulante Peritonealdialyse (CAPD) zur Detektion eines Behandlungsstatus, insbesondere zur Detektion eines Phasenabschnitts und/oder eines Phasenübergangs bei der Peritonealdialyse.

Dialyseverfahren sind in der medizinischen Therapie weit verbreitet und dienen der Behandlung von verschiedenen Krankheiten und Störungen. Dabei werden Dialyseverfahren beispielsweise bei Patienten mit akuter oder chronischer Niereninsuffizienz in verschiedenen Krankheitsstadien zur Blutreinigung eingesetzt.

Grundsätzlich unterscheidet man bei den Dialyseverfahren zwischen den extrakorporalen (außerhalb des Körpers) und intrakorporalen (innerhalb des Körpers) Verfahren. Zu den extrakorporalen Verfahren zählen die Hämodialyse, Hämofiltration sowie Hämodiafiltration und als intrakorporales Verfahren die Peritonealdialyse.

Während beispielsweise bei der Hämodialyse das Blut über einen Filter mit einer speziellen Membran gereinigt wird, dient bei der Peritonealdialyse das Peritoneum, welches auch als Bauchfell bezeichnet wird, als körpereigene Filtermembran. Bei der Bauchfelldialyse wird eine Dialyselösung in den Bauchraum eingeleitet, welche die Stoffwechselprodukte aufnimmt und nach einer gewissen Verweilzeit wieder aus dem Bauchraum entnommen wird.

Zur Durchführung der Peritonealdialyse stehen sowohl manuelle als auch automatische Methoden zur Verfügung. Bei der ambulanten kontinuierlichen Peritonealdialyse (CAPD) handelt es sich um ein manuelles Verfahren, bei dem der Patient selbst mehrmals täglich die Dialyselösung im Bauchraum austauscht. Bei der automatischen Peritonealdialyse (APD) erfolgt der Wechsel der Dialyselösung über ein Gerät, den sogenannten Cycler. Die APD wird meistens nachts durchgeführt, während der Patient schläft.

Wie bereits erwähnt, handelt es sich bei der CAPD um ein manuelles Verfahren, welches gravimetrisch betrieben werden kann. Aber auch die APD-Cycler können als gravimetrisch arbeitende Cycler konzipiert sein.

Zur Steuerung, Überwachung und Datenerfassung der PD-Behandlung sind die APD-Cycler beispielsweise mit einer umfangreichen Mechanik, wie Pumpen, Ventilen, Motoren und Elektronik, wie Sensoren und Datenverarbeitungseinrichtungen ausgestattet. Dabei beruht die Steuerung des Cyclers und insbesondere die Ermittlung eines aktuellen Behandlungsstatus auf dem Zusammenspiel der oben genannten Komponenten zur Bestimmung von behandlungsspezifischen Messdaten, wie Volumen, Druck, Gewicht, Flussraten und im Abgleich mit einer voreingestellten Behandlungsverschreibung.

Die ermittelten Messdaten können in einer Datenverarbeitungseinrichtung derart aufgearbeitet werden, dass sich diverse Dialyseparameter ermitteln lassen, die darüber hinaus auch Aufschluss über den Behandlungsablauf und das Behandlungsziel geben können.

In der US 5,445,610 ist ein gravimetrischer PD-Cycler beschrieben, welcher unter anderem über eine Wägezelle verfügt. Die, mit der Wägezelle erzielten Gewichtsmessungen werden in einem Computer mit voreingestellten Daten abgeglichen und mittels eines Controllers in entsprechende Volumina umgerechnet. Dies ermöglicht die automatische Steuerung der Ventile und die Festlegung der Füll-, Dwell- sowie Drainanteile für jeden einzelnen Behandlungszyklus. Darüber hinaus lässt sich auch die Ultrafiltrationsmenge ermitteln.

Die EP 0 097 432 beschreibt ebenfalls einen gravimetrisch arbeitenden PD-Cycler, welcher über zwei Waagen verfügt. Zur Erreichung eines definierten Füllvolumens wird das Beutelgewicht der ersten Waage solange mit einem, aus der Therapieverschreibung, voreingestellten Wert abgeglichen, bis dieser erreicht ist. Mit dem Erreichen des voreingestellten Gewichts auf der Waage werden gleichzeitig die Ventile automatisch aktiviert und gesteuert. Das Draingewicht wird über die zweite Waage erfasst. Mit Hilfe eines Computers lässt sich das Ultrafiltrationsgewicht und damit auch das Ultrafiltrationsvolumen aus dem Füll- und Draingewicht ermitteln.

Die CA 2 223 846 beschreibt ein Dialysegerät mit einer Waage sowie einem Prozessor zur Erfassung der mit der Waage ermittelten Gewichtsdaten.

In der WO 96/37243 ist eine tragbare Pumpe offenbart, welche mit verschiedenen Sensoren ausgestattet sein kann um verschiedene patientenspezifische Parameter erfassen zu können.

CAPD-Systeme, die rein gravimetrisch arbeiten und manuell bedient werden, erfordern keine gerätetechnisch aufwändige Ausstattung. Daher sind diese Systeme einfach und kostengünstig herzustellen und zu betreiben sowie nahezu störungs- und wartungsfrei gegenüber den übrigen Dialyseverfahren.

Diese Systeme erfordern allerdings ein hohes Maß an Eigenverantwortung und Disziplin seitens des Patienten bei der Durchführung der Behandlung und der Dokumentation.

Es ist daher Aufgabe der Erfindung, ein gravimetrisch arbeitendes und manuell bedienbares CAPD-System bereitzustellen, welches mit geringem technischen Aufwand den Patienten über den aktuellen Behandlungsstatus, insbesondere einen Phasenabschnitt und/oder einen Phasenübergang während einem Zyklus bei der PD-Behandlung bzw. mittels Hinweisen oder Anweisungen über den weiteren Behandlungsverlauf, informiert

Die Aufgabe wird gelöst durch den Gegenstand des Anspruchs 1. Dabei ist eine Sensorik vorgesehen, die an ein gravimetrisch arbeitendes und manuell zu bedienendes CAPD-System angebracht ist und mit der eine Veränderung gemessen werden kann, wobei die Sensorik zwei Gewichtssensoren umfasst und mit einer Auswerteeinheit verbunden ist, wobei die Auswerteeinheit derart angepasst ist um die mit den beiden Gewichtsensoren ermittelten Messdaten umzuwandeln um einen Phasenabschnitt und/oder Phasenübergang zu einem bestimmten Zeitpunkt zu bestimmen. Damit kann auf den Status einer Dialysebehandlung zu einem bestimmten Zeitpunkt geschlossen und die Daten auf einem Speichermedium gespeichert werden.

Die Aufgabe wird weiterhin durch ein Verfahren, zur Messung einer Veränderung mittels einer Sensorik, die an ein gravimetrisch arbeitendes und manuell zu bedienendes CAPD-System angebracht ist, gemäß Anspruch 11 gelöst.

Weiterhin wird die Aufgabe durch ein gravimetrisch arbeitendes und manuell bedienbares CAPD-System mit einer Sensorik zur Messung einer Veränderung gemäß Anspruch 24 gelöst.

Weitere vorteilhafte Ausführungsformen der Erfindung ergeben sich aus den Unteransprüchen.

Bei den CAPD-Systemen, wie sie im Sinne der Erfindung verwendet werden, handelt es sich um Systeme, die nach einem rein gravimetrischen Prinzip arbeiten und manuell bedient werden, enthaltend mindestens einen Dialysebeutel, der vorzugsweise bereits gebrauchsfertig mit Dialyselösung befüllt ist, mindestens einem Drainagebeutel, einer Vorrichtung zum Steuern eines Fluidverlaufs, einem Schlauchsystem zum Verbinden der Beutel mit einem Katheteranschluss zum Patienten und einer Sensorik.

Für die Steuerung der einzelnen Fluidwege, wie das Ablassen des verbrauchten Dialysats aus dem Patienten, das Spülen des Schlauchsystems und das Befüllen des Peritoneums mit Dialysat, erfolgt gemäß der Erfindung mit Hilfe von manuell bedienbaren Ventilen oder Klemmvorrichtungen. Dabei können die Ventile als Ein- oder Mehrwegventile und die Klemmvorrichtungen zum Beispiel als Schlauchklemmen ausgebildet sein. Die Fluidsteuerung wird manuell von dem Patienten oder Anwender vorgenommen.

Die Sensorik gemäß der Erfindung, kann mindestens zwei Sensoren umfassen, die mit einer Auswerteeinheit, einer Anzeigeeinheit und/oder einer Zeitmesseinrichtung zum Beispiel in Form eines Timers, einer Stoppuhr oder einer Uhr mit Zeit- und Datumsanzeige verbunden sein können und eine Verbindung zu einem Speichermedium aufweisen. Die Sensorik ist dabei an einem gravimetrisch arbeitenden und manuell bedienbarem CAPD-System angebracht.

Bei den Sensoren, gemäß der Erfindung, handelt es sich um Gewichtssensoren.

Die Auswerteeinheit dient im Sinne der Erfindung zur Aufnahme der Messdaten bzw. gemessenen Signale und zum Abgleich derselben, so dass insbesondere die einzelnen Phasenabschnitte und/oder Phasenübergänge bestimmt werden können und somit eine Aussage über den Status einer Dialysebehandlung zu einem bestimmten Zeitpunkt zulassen, welcher über eine Anzeigeeinheit ausgegeben und die Messdaten oder gemessenen Signale auf einem Speichermedium festgehalten werden können. Vorteilhafterweise ist eine Zeitmesseinrichtung in der Auswerteeinheit integriert.

Im Folgenden wird der Gegenstand der Erfindung unter Bezugnahme auf die Zeichnungen näher erläutert.
Fig. 1 zeigt den schematischen Aufbau eines erfindungsgemäßen gravimetrisch arbeitenden und manuell bedienbaren CAPD-Systems.
Fig. 2 zeigt eine graphische Darstellung der einzelnen Phasenabschnitte und Phasenübergänge während eines Zyklus einer CAPD-Behandlung.

Üblicherweise werden bei der Peritonealdialyse mehrere Zyklen durchlaufen, die aus drei Phasen bestehen. Die Dialysebehandlung startet in einem ersten Schritt mit der Drainagephase, bei der das verbrauchte Dialysat aus dem Peritoneum abgelassen wird. In einem 2. Schritt folgt die Prime- oder Spülphase um mit dem Dialysat aus dem Dialysatbeutel Luft aus dem Schlauchsystem zu spülen. Die Lösung zum Spülen wird mittels der Vorrichtung zum Steuern eines Fluidverlaufs direkt in den Drainagebeutel geleitet. Mit dem 3. Schritt erfolgt die Fill-Phase, oder auch Einfüllphase genannt, zum Befüllen des Peritoneums mit frischer Dialyselösung. Entsprechend der Behandlungsverschreibung verbleibt die Lösung über einen gewissen Zeitraum im Peritoneum bevor der nächste Zyklus wieder mit der 1. Phase beginnt. Dieser Prozess wird entsprechend der Behandlungsverschreibung mehrmals täglich wiederholt.

Da sich die Peritonealdialyse, insbesondere die kontinuierliche ambulante Peritonealdialyse, aufgrund ihrer einfachen Bedienbarkeit und geringen Stör- und Wartungsanfälligkeit sehr gut für den Heimdialysebereich eignet, erfordert sie dennoch eine gute Schulung und ein hohes Maß an Eigenverantwortlichkeit und Disziplin des Patienten bei der Durchführung der Dialysebehandlung und Dokumentation. Daher ist es von Vorteil wenn bei einer Peritonealdialyse der aktuelle Behandlungsstatus, insbesondere die Phasenabschnitte und/oder Phasenübergänge angezeigt werden können. Besonders vorteilhaft ist es dabei, wenn der Patient, auf Basis der mit der Sensorik ermittelten Messdaten oder Signalen, einen Hinweis erhält, wie in der Behandlung weiter zu verfahren ist.

Die Information über einen aktuellen Behandlungsabschnitt erlaubt es dem Patienten, einen schnelleren und besseren Überblick über den Behandlungsstatus zu erhalten, insbesondere dann, wenn sein Eingreifen erforderlich ist. Beispielsweise kann er über eine Anzeigeeinheit informiert werden, dass er sich bei seiner Behandlung in der Drain- oder Drainagephase befindet, bzw. diese noch nicht abgeschlossen ist und/oder es kann das Ende der Drainphase angezeigt werden. Dadurch kann vermieden werden, dass der Patient zu früh mit der Einfüllphase beginnt und damit das Füllvolumen im Peritoneum über ein verträgliches Maß hinaus ansteigt und zu einer Überfüllung des Patienten führt. Am Ende der Drainphase kann dann ein Hinweis erfolgen, dass nun die Primephase vom Patienten zu starten ist. Weiterhin kann er am Ende der Primephase aufgefordert werden, diese zu beenden und die Einfüllphase zu starten. Auch auf das Ende der Einfüllphase kann er mittels der Anzeigeeinheit aufmerksam gemacht werden, in dem zum Beispiel eine schriftliche Anzeige das Einfüllende kenntlich macht oder eine schriftliche Anzeige den Patienten oder Anwender auffordert die Dekonnektion vorzunehmen um den Behandlungszyklus zu beenden. In einer besonderen Ausführungsform kann das Ende einer jeden Phase durch ein akustisches Signal angezeigt werden, insbesondere beim Erreichen des Soll-Einfüllgewichts /-volumens um das Ende des Behandlungszyklus zu signalisieren.

Die Angabe der verschiedenen Phasenabschnitte und/oder Phasenübergänge während der Behandlung bietet dem Patienten einen verbesserten Komfort und eine höhere Sicherheit ohne dabei eine aufwändige Technik bereitstellen zu müssen. So ist es, mittels der Sensorik möglich, den Behandlungsstatus zu erkennen, ohne dass eine technisch aufwändige Aktorik erforderlich ist. Des Weiteren ist es gerade in der Primephase von essentieller Bedeutung, dass diese ausreichend lange durchgeführt wird um die im Schlauchsystem enthaltene Luft vollständig zu entfernen. Wird die Primephase zu früh beendet, gelangt die Luft aus dem Schlauchsystem in das Peritoneum was zu dem Krankheitsbild der Pneumoperitonitis führen kann. Wird dagegen das Schlauchsystem zu lange gespült, besteht die Gefahr, dass für die eigentliche Behandlung nicht mehr ausreichend Dialysat zur Verfügung steht. Bei den, aus dem Stand der Technik bekannten, gravimetrisch arbeitenden und manuell bedienbaren CAPD-Systemen, muss der Patient bzw. Anwender den geeigneten Zeitpunkt für das Ende der Primephase selbst erkennen. Durch eine Aufforderung, die Primephase zu beenden kann er zum richtigen Zeitpunkt auf die Überleitung zum nächsten Behandlungsschritt aufmerksam gemacht werden.

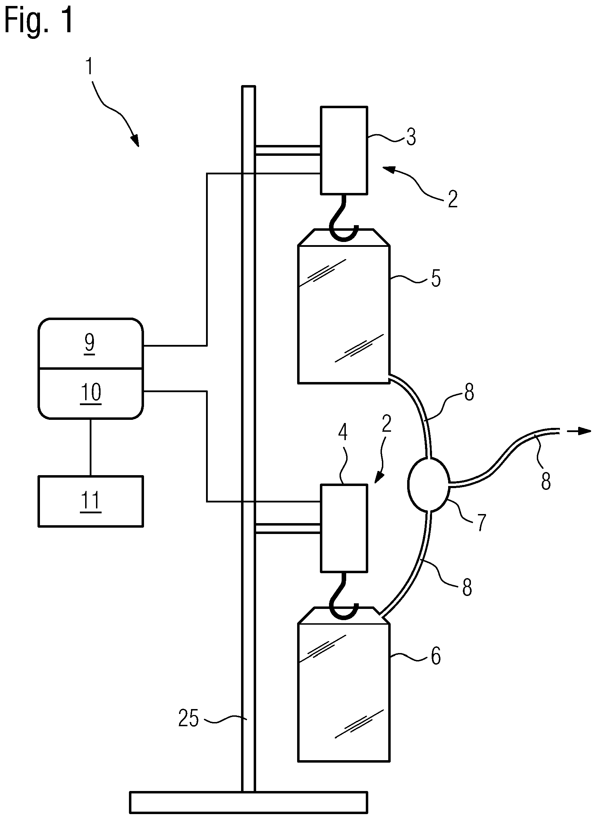

Zur Veranschaulichung ist in Fig. 1 beispielhaft der Aufbau eines gravimetrisch arbeitenden und manuell bedienbaren CAPD-Systems (1) schematisch dargestellt, an das eine Sensorik (2), angebracht ist und mit der eine Veränderung gemessen werden kann, wobei die Veränderung auf den Status einer Dialysebehandlung zu einem bestimmten Zeitpunkt schließen lässt, und die Daten auf einem Speichermedium (11) festgehalten werden können. Die Sensorik (2) zeichnet sich dadurch aus, dass sie zur Bestimmung einer Flussratenmessung, Füllstandsmessung, Druckmessung, Volumenmessung und/oder einer Gewichtsmessung geeignet ist. Hierzu kann die Sensorik (2) mit optischen Sensoren, Drucksensoren, Durchflusssensoren, Volumensensoren und/oder Gewichtssensoren ausgestattet sein. Des Weiteren umfasst die Sensorik (2) mindestens zwei Sensoren (3, 4). Dabei ist ein Dialysatbeutel (5) über einen ersten Sensor (3) an einer Haltevorrichtung (25) befestigt. Unterhalb des Dialysatbeutels (5) befindet sich ein Drainagebeutel (6), der über einen zweiten Sensor (4) ebenfalls an der Haltevorrichtung (25) befestigt ist. In einer bevorzugten Ausführungsform handelt es sich bei den Sensoren (3, 4) um Gewichtssensoren, wie sie beispielsweise in elektronischen Waagen verwendet werden. Die Fluidströme werden über eine Fluidsteuerungsvorrichtung (7) entsprechend der jeweiligen Phasen geregelt. Die Fluidsteuerungsvorrichtung (7) stellt über ein Schlauchsystem (8) den Fluidfluss zwischen dem Dialysatbeutel (5), dem Drainagebeutel (6) und dem Bauchanschluss zum Patienten dar. Die Sensorik (2) kann dabei noch über ein Speichermedium (11) verfügen, auf dem die Messdaten bzw. gemessenen Signale festgehalten werden können.

Die Sensorik (2) ist weiterhin mit einer Auswerteeinheit (9) verbunden. Die Datenerfassung erfolgt mittels der Sensoren (3, 4), wobei zur Bestimmung der Veränderung eines Status einer Dialysebehandlung die Messdaten oder Signale von mindestens zwei Sensoren (3, 4) an die Auswerteeinheit (9) weitergeleitet werden. Dabei können die Messdaten der beiden Sensoren (3, 4) zu einem bestimmten Zeitpunkt gegeneinander abgeglichen werden. Vorteilhafterweise können die Messdaten von den beiden Sensoren (3, 4) zum gleichen Zeitpunkt erfasst und mittels einer Auswerteeinheit (9) zeitgleich gegeneinander abgeglichen werden. Besonders bevorzugt ist darüber hinaus eine Aufnahme der Messdaten der mindestens zwei Sensoren (3, 4), welche mit Hilfe der Auswerteeinheit kontinuierlich gegeneinander abgeglichen werden können. Die, auf Basis der Sensoren, ermittelten Messdaten oder Signale können in einer Auswerteeinheit (9) derart umgewandelt werden, dass diese als ein Phasenabschnitt und/oder ein Phasenübergang zu einem bestimmten Zeitpunkt einer Dialysebehandlung auf einer Anzeigeeinheit (10) ausgegeben werden können. In einer weiteren Ausführungsform kann über die Auswerteeinheit (9) auch ein Hinweis oder eine Anweisung auf der Anzeigeeinheit (10) ausgegeben werden, wie die weitere Behandlung fortzusetzen ist. Hierzu ist die Sensorik (2), mit einer Zeitmesseinrichtung (nicht dargestellt), zum Beispiel in Form eines Timers oder einer Stoppuhr gekoppelt, um die Phasenabschnitte und/oder Phasenübergänge in Abhängigkeit von der Zeit t ermitteln zu können. Vorteilhafterweise ist die Zeitmesseinrichtung in der Auswerteeinheit (9) integriert.

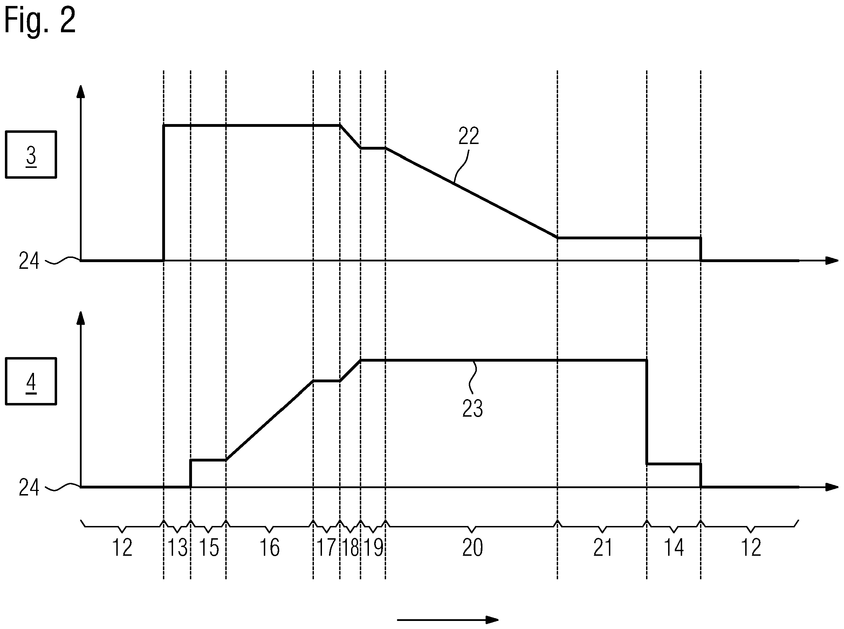

Mit der Auswerteeinheit (9) lassen sich, wie in Fig. 2 beispielhaft dargestellt, folgende Phasenabschnitte und/oder Phasenübergänge bestimmen. Vor Beginn der Behandlung, d. h. wenn weder der Dialysat- noch der Drainagebeutel angehängt sind, befindet sich das CAPD-System in einem unbeladenen Zustand (12). Ein unbeladener Zustand (12) liegt auch dann vor, wenn die beiden Beutel (5, 6) nach der Behandlung abgenommen wurden. Mit dem An- bzw. Abhängen eines oder mehrerer Dialysat- oder Drainagebeutel (5, 6) ändern sich die, mittels der Sensoren (3, 4), gemessenen Signale, so dass sich anhand der Veränderung der Daten die Belade- und Entladephasen (13, 14) bestimmen lassen. Nach der Beladung erfolgt keine weitere Datenänderung. Das System befindet sich in einem ersten Phasenübergang (15). Mittels der Sensoren (3, 4) ist es auch möglich, zu prüfen, ob die Beladung mit den Beuteln (5, 6) korrekt ausgeführt wurde, so dass auf diesem Weg eine Fehlbedienung ausgeschlossen werden kann. Mit dem Ablassen des verbrauchten Dialysats aus dem Peritoneum des Patienten wird die Drainphase (16) eingeleitet, die sich ebenfalls aus dem Verhältnis der Messdaten der Sensoren (3, 4) zueinander ermitteln lässt. Nachdem das verbrauchte Dialysat aus dem Peritoneum abgeflossen ist, wird ein weiterer konstanter Zustand erreicht, da an beiden Sensoren (3, 4) keine Signaländerung zu verzeichnen ist, so dass nun ein zweiter Phasenübergang (17) erreicht ist. Daran schließt sich die Spül- oder Primephase (18) an, wobei wiederum eine Signaländerung an beiden Sensoren (3, 4) zu erkennen ist. Im Abschluss an die Spülphase (18) wird ein dritter Phasenübergang (19) erreicht, in der sich die Messdaten der mindestens zwei Sensoren (3, 4) zueinander ebenfalls nicht ändern. Mit dem Einleiten von Dialysat in den Patienten beginnt die Einfüllphase (20), die dann abgeschlossen ist, sobald das gesamte Dialysat aus dem Dialysatbeutel (5) abgeflossen bzw. das vom Arzt verschriebene Füllvolumen erreicht ist und sich der Messwert des ersten Sensors (3) nicht mehr verändert. Das Füllvolumen wird im vorliegenden Beispiel mittels der Sensoren als Füllgewicht erfasst und kann mittels einer Auswerteeinheit in eine Volumenangabe umgewandelt und angezeigt werden. Danach beginnt die Dwellphase, während dieser das Dialysat für eine vorgegebene Zeit im Peritoneum verbleibt. Die Dwellphase ist hier als vierter Phasenübergang (21) zu verstehen, da während dieser Phase das Dialysat im Patienten verbleibt und kein Fluidtransfer und somit keine Signaländerung an den Sensoren erfolgt. Mit dem Endladevorgang, d. h. der Abnahme der Beutel, wird, wie bereits oben beschrieben, wieder ein 0-Punkt oder Ausgangswert an den Sensoren (3, 4) erreicht. Die Phasenübergänge zeichnen sich insbesondere dadurch aus, dass über eine Zeit t keine Signaländerung an den beiden Sensoren (3, 4) erfasst wird.

Die einzelnen Phasenübergänge liegen zwischen den oben beschriebenen Phasen und sind in Fig. 2 als senkrechte gestrichelte Linien dargestellt. Sie können ebenfalls mittels der Auswerteeinheit (9) anhand der Änderung der Messdaten bzw. Signale bestimmt werden. Dabei können folgende Phasenübergänge ermittelt werden:
- Ausgehend von dem Beginn des Beladevorgangs (13) des CAPD-Systems (1) mit einem oder mehreren Dialysat- bzw. Drainagebeutel(n) (5, 6) bis zum Erreichen eines 1. Phasenübergangs (15), wobei der 1. Phasenübergang (15) zwischen dem Ende des Beladevorgangs (13) und dem Beginn der Drainphase (16) liegt,
- einem 2. Phasenübergang (17) zwischen dem Ende der Drainphase (16) und dem Beginn der Spül- bzw. Primephase (18),
- einem 3. Phasenübergang (19) zwischen dem Ende der Spülphase (18) und dem Beginn der Einfüllphase (20) und
- einem 4. Phasenübergang (21) zwischen dem Ende der Einfüllphase (20) und der Entladephase (14) der oder des Dialyse- bzw. Drainagebeutel(s).

Vorzugsweise erscheint die Anzeige der Phasenabschnitte, Phasenübergänge und/oder Messdaten in einer nummerischen, schriftlichen oder symbolischen Darstellung auf der Anzeigeeinheit (10), zum Beispiel in Form eines Displays, Bildschirms oder Monitors.

Die bei der Behandlung ermittelten Messdaten bzw. Signale zur Bestimmung einer aktuellen Behandlungsphase bzw. Phasenabschnitts und/oder eines Phasenübergangs können zur Speicherung und einer möglichen Weiterverarbeitung auf einem Speichermedium (11) festgehalten werden. Dies erleichtert dem Patienten die Dokumentation seiner Daten. Die Daten müssen dann nicht mehr handschriftlich in einem Datenblatt erfasst werden und können zur weiteren Auswertung einer Datenverarbeitungseinrichtung zugeführt werden. Somit kann auch der behandelnde Arzt auf eine fundierte Datenlage zurückgreifen, anhand dessen er den Behandlungsverlauf nachverfolgen kann. Darüber hinaus lassen sich auch therapierelevante Daten unter Verwendung gespeicherter Daten aus vorangegangenen CAPD-Behandlungen bestimmen. So kann zum Beispiel mittels des aktuellen Drainagegewichts /-volumens und dem gespeicherten Füllgewichts- /volumen, das Ultrafiltrationsvolumen berechnet werden. Weiterhin lässt sich aus den Messwerten der Beutelwaagen in der Drain- Spül- und/oder Einfüllphase der Fluidfluss (Volumenänderung pro Zeit) ermitteln. Insbesondere lässt die Dauer der Drain- oder Einfüllphase Rückschlüsse auf den Zustand des Patientenkatheters zu. Für den Fall, dass der Patientenkatheter verstopft oder der Katheterschlauch abgeknickt ist, führt dies zu einer deutlich verlängerten Drain- und/oder Einfüllphase, im Vergleich zu vorherigen Behandlungen oder der in der Verschreibung vorgesehenen Zeiten. Durch einen Abgleich der Zeitdauer mit den Drain- und/oder Einfüllzeiten aus früheren Behandlungen oder den, in der Behandlungsverschreibung, festgelegten Zeiten können solche Fehlerquellen erkannt und durch einen entsprechenden Hinweis auf der Anzeigeeinheit (10) entsprechende Gegenmaßnahmen durch den Patient oder Anwender eingeleitet werden.

Mit der Erkennung der einzelnen Phasenabschnitte und/oder Phasenübergänge lassen sich darüber hinaus die verschiedenen Behandlungsvolumina, wie das Drainage-, Einfüll- und Spülvolumen berechnen. Diese können aus den Messdaten der wenigstens zwei Sensoren, insbesondere der Gewichtssensoren der Beutelwaagen bestimmt werden, die zwischen den Phasenübergängen gemessen, und in einer Auswerteeinheit in eine Volumenangabe umgewandelt werden. So kann zum Beispiel das Einfüll-Volumen aus den Messwerten von dem Phasenübergang zwischen dem Einfüllstart und dem Phasenübergang am Einfüllende ermittelt werden.

Die Berechnung und Weiterverarbeitung einzelner Behandlungsparameter kann sowohl mit der, an die Sensorik (2) angekoppelten, Auswerteeinheit (9) als auch in externen Datenverarbeitungsvorrichtungen durchgeführt werden.

Zur Datenablage können interne Speichermedien (11) in Form einer internen Festplatte vorgesehen sein oder die Daten vorzugsweise auf einem austauschbaren Speichermedium, wie beispielsweise einem leichttransportablem Speichermedium in Form einer Chipkarte, eines USB-Sticks, einer wiederbeschreibbaren CD oder ähnlichen Datenträgern abgelegt werden. Besonders bevorzugt können die Daten auch auf externen Speichermedien, zum Beispiel in Form einer externen Festplatte einer externen Speicherkarte oder einem Daten-Hub in einem separaten Datenverarbeitungsgerät, übertragen werden. Dabei können die Daten kabelgebunden oder drahtlos beispielsweise an ein Handy, einen PC, Laptop, Tablet oder dergleichen übertragen und dort weiterverarbeitet werden. Bei einer drahtlosen Datenübertragung auf ein externes Speichermedium (11) können die Messdaten zum Beispiel mittels Bluetooth, W-Lan, ZigBee, Infrarot, optische Übertragung mittels QR-Code, akustischer Datenübertragung über Tonabfolge, Nahfeldkommunikation oder anderen geeigneten drahtlosen Kommunikationseinrichtungen übertragen werden.

Dabei ist es selbstverständlich, dass die Speichermedien (11) über die entsprechenden Zugänge und Anschlüsse sowie Empfangsvorrichtungen zur Übertragung und Speicherung der Messdaten verfügen.

In einer besonders bevorzugten Ausführungsform können die, während einer Behandlung von einer Sensorik (2) ermittelten und auf einem Speichermedium (11) festgehaltenen, Messdaten mittels einer internen Auswerteeinheit (9) oder einer externen Datenverarbeitungsvorrichtung zur Berechnung von einzelnen Behandlungsparametern herangezogen werden und/oder durch den Abgleich mit Messdaten aus vorangegangenen Behandlungen abgeglichen werden. So können die Messdaten beispielsweise bezüglich des Ultrafiltrationsvolumens mit denen, in der Behandlungsverschreibung, hinterlegten Daten oder den Daten aus vorangegangenen Behandlungen abgeglichen werden um Veränderungen bzw. Abweichungen vom angestrebten Therapieziel erkennen zu können. Weiterhin können anhand der gespeicherten Daten aus vorherigen Behandlungen mögliche Trends hinsichtlich verschiedener Dialyseparameter (wie zum Beispiel Einfüllvolumen, Ultrafiltrationsvolumen, Dwellzeit) abgeleitet werden, welche zur Anpassung einer künftigen Behandlungsverschreibung herangezogen werden können. Die Messdaten können ebenfalls zur Fehlererkennung, wie oben erwähnt, verwendet werden, so dass zum Beispiel ein nicht korrekter Beladezustand, ein Katheterfunktionszustand in Form eines verstopfter Katheranschlusses oder ein geknickter Katheterschlauch erkannt werden können.

In einer weiteren bevorzugten Ausführungsform kann das gravimetrisch arbeitende und manuell bedienbare CAPD-System (1) weiterhin zur Bestimmung der Transportkapazität des Pertioneums (PDC = Peritoneal Dialysis Capacity) herangezogen werden. Für eine PDC-Bestimmung ist unter anderem die Ermittlung des exakten Ultrafiltrationsvolumens relevant. Dies ergibt sich aus der Differenzmessung des frischen Dialysats im Dialysatbeutel und dem nach der Dwellzeit abgelassenen verbrauchten Dialysats, einschließlich der vom Körper drainierten Flüssigkeit. Da die Dialysatbeutel in der Regel über ein höheres als nominal angegebenes Füllvolumen verfügen, um dieses nominale Füllvolumen auch am Ende der Lagerzeit noch gewährleisten zu können, ist das tatsächliche Dialysatvolumen zum Zeitpunkt der Applikation nicht genau bekannt. Mittels der Sensoren (3, 4) lässt sich das Gewicht der Dialysat- und Drainagebeutel (5, 6) im gefüllten und entleerten Zustand, einschließlich des Schlauchsets (8) mit der Fluidsteuerungsvorrichtung (7), bestimmen und daraus das exakte Ultrafiltrationsgewicht errechnen. Das Schlauchset (8) mit der Fluidsteuerungsvorrichtung (7) wird üblicherweise zu Beginn der Behandlung an der Halterung (25) befestigt und verbleibt auch während der Behandlung dort, so dass sich deren Gewichte anteilig auf beide Sensoren (3, 4) aufteilen. Durch einen Vergleich der gespeicherten Ultrafiltrationsgewichte aus vorangegangenen Behandlungen können Veränderungen im Ultrafiltrationsverhalten erkannt werden. Anhand der für das Dialysat angegebenen Dichte kann das Ultrafiltrationsvolumen aus dem Differenzgewicht berechnet werden.

In einem bevorzugten Ausführungsbeispiel, wie in Fig. 2 graphisch dargestellt, wird ein Verfahren beschrieben zur Messung einer Veränderung mittels einer Sensorik (2), die an ein gravimetrisch arbeitendes und manuell bedienbares CAPD-System (1) angebracht ist, wobei die Sensorik (2) umfassend zwei Gewichtssensoren (3, 4) und eine Auswerteeinheit (9), welche die mit den Gewichtssensoren ermittelten Messdaten umwandelt um einen Phasenabschnitt und/oder Phasenübergang zu einem bestimmten Zeitpunkt zu bestimmen. Damit kann auf den Status einer Dialysebehandlung zu einem bestimmten Zeitpunkt geschlossen und diese Daten auf einem Speichermedium (11) festgehalten werden.
Im vorliegenden Beispiel erfolgt die Datenerfassung mit zwei Sensoren (3, 4), wobei vorzugsweise Gewichtssensoren, wie sie bei elektronischen Waagen verwendet werden, zum Einsatz kommen. Die, zu bestimmten Zeitpunkten, von einem ersten und einem zweiten Sensor (3, 4) gemessenen Daten bzw. Signale werden mittels einer Auswerteeinheit (9) gegeneinander abgeglichen. Dabei ist es vorteilhaft, wenn die Daten zeitgleich aufgenommen und gegeneinander abgeglichen werden. In einer besonders bevorzugten Ausführungsform erhält man bei einer kontinuierlichen Messung der Beutelgewichte mittels der beiden Sensoren (3, 4) und deren kontinuierlichen Abgleich über die Zeit sowohl für den Dialysatbeutel (5) als auch für den Drainagebeutel (6) jeweils einen Streckenverlauf (22, 23), wobei die beiden Streckenverläufe (22, 23) gegeneinander abgeglichen und in einer Auswerteeinheit (9) umgewandelt werden, um für einen bestimmten Zeitpunkt einen Phasenabschnitt und/oder Phasenübergang auf einer Anzeigeeinheit (10) ausgeben zu können. Somit lässt sich durch die Auswertung der zeitlichen Änderung der Messdaten jeweils für ein entsprechendes Zeitfenster ein Signaltrend (z.B. Signal bleibt gleich, Signal nimmt ab oder Signal nimmt zu) ermitteln. Weiterhin lässt sich ein Signaltrend für ein ausgewähltes Zeitfenster anhand eines Streckenverlaufs (22, 23) quantifizieren. Am Beginn der Zeitachse befinden sich die beiden Sensoren (3, 4) an einem sogenannten 0-Punkt oder Ausgangswert (24), das bedeutet, es ist noch keine Signaländerung zu verzeichnen, da das CAPD-System noch nicht mit den Beuteln (5, 6) beaufschlagt wurde. Während dem Beladevorgang mit dem befüllten Dialysatbeutel ist eine sprunghafte Änderung im ersten Streckenverlauf (22) zu beobachten und nur eine geringe Signaländerung durch das Anhängen des noch leeren Drainagebeutels im zweiten Streckenverlauf (23). Durch die sprunghafte und versetzte Veränderung der Messsignale erkennt die Auswerteeinheit (9), dass sich das gravimetrisch arbeitende und manuell bedienbare CAPD-System (1) in der Beladephase (13) befindet. Da nach dem Beladevorgang keine Signaländerung mehr bestimmbar ist, erkennt die Auswerteeinheit (9), dass sich das System in einem ersten Phasenübergang (15) befindet. Aufgrund der vorangegangenen Messdaten kann mittels der Auswerteeinheit (9) der Sensorik (2) vorzugsweise ein Hinweis auf einer Anzeigeeinheit (10) ausgegeben werden, die den Patienten auffordert, eine Drainage einzuleiten. Kommt es im weiteren Verlauf der Behandlung nun zu einem Anstieg des zweiten Streckenverlaufs (23) und ist im ersten Streckenverlauf (22) keine Signaländerung zu verzeichnen, erkennt die Auswerteeinheit (9), dass sich das CAPD-System in der Drainphase befindet. Auf der Anzeigeeinheit (10) kann nun der aktuelle Phasenabschnitt der "Drainage" angezeigt werden. Sobald weder in dem ersten noch in dem zweiten Streckenverlauf (22, 23) eine Signaländerung zu messen ist, erkennt die Auswerteeinheit (9), dass der Drainagevorgang abgeschlossen und ein zweiter Phasenübergang (17) erreicht ist. Über die Anzeigeeinheit (10) kann nun eine Aufforderung, beispielsweise zum Starten eines Spülvorgangs ausgegeben werden, welche während der Spülphase (18) optisch auf der Anzeigeeinheit (10) angezeigt werden kann. Die Spülphase (18) wird von der Auswerteeinheit (9) dadurch festgestellt, dass das Signal am ersten Sensor (22) abnimmt und das Signal am zweiten Sensor (23) zunimmt. Da bei einem manuell betriebenen CAPD-System (1) der Patient die Einleitung und Durchführung der einzelnen Phasen selbsttätig vornimmt, wird die Spülphase (18) mit der Luftfreiheit des Schlauchsystems durch den Patienten beendet. Es kommt zu keiner weiteren Signaländerung und ein dritter Phasenübergang (19) ist erreicht. Auf das Ende der Spülphase (18) (oder auch der übrigen Phasenabschnitte und Phasenübergänge) kann der Patient oder Anwender zum Beispiel auch auf Basis des Fluidflusses oder des Fluidvolumens aufmerksam gemacht werden, in dem er durch eine optische Anzeige auf der Anzeigeeinheit oder ein akustisches Signal aufgefordert wird, die Spülphase (18) zu beenden. Aus den vorangegangen Messdaten bzw. Signaltrends der mindestens zwei Sensoren (3, 4) ist es mittels der Auswerteeinheit (9) möglich, die entsprechenden Phasenabschnitte und/oder Phasenübergänge abzuleiten und in diesem Behandlungsstadium eine Aufforderung an den Patienten auszugeben, zum Starten der Einfüll- oder Fillphase (18). Durch eine kontinuierliche Aufnahme der Messdaten der Sensoren (3, 4) lässt sich in der Einfüllphase für den ersten Sensor (22) ein abnehmender Signaltrend erkennen, während sich das Signal am zweiten Sensor (23) nicht verändert, da das Dialysat in das Peritoneum des Patienten geleitet wird. Aufgrund des Abgleichs der beiden Streckenverläufe (22, 23) wird von der Auswerteeinheit (9) die Einfüllphase (18) erkannt und kann ebenfalls als Information über den aktuellen Status von der Anzeigeeinheit (10) ausgegeben werden. Das Ende der Einfüllphase (18) ist dann erreicht, wenn von dem ersten Sensor (22) ein konstantes Signal empfangen wird, welches direkt in die Dwell- oder Verweilphase übergeht. Die Dwellphase stellt damit einen vierten Phasenübergang (21) dar, da hier sowohl am ersten als auch am zweiten Sensor (3, 4) keine Signaländerung zu beobachten ist. Mit Erreichen des vierten Phasenübergangs (21) kann der Patient oder Anwender über die Anzeigeeinheit (10) informiert werden, dass nun die Dekonnektion erfolgen kann. Durch die Abnahme der Beutel (5, 6) kommt es zu einer geringen Signaländerung am ersten Sensor (3) des Dialysatbeutel (5) und zu einer sehr viel größeren Signaländerung am zweiten Sensor (4) des Drainagebeutels (6), da dieser nun gefüllt ist. Durch den Abgleich der beiden Signaltrends nach dem Endladevorgang (14) und der vorangegangenen Phaseninformationen erkennt die Auswerteeinheit (9) das Erreichen eines 0-Punkts oder Ausgangswerts (24) und damit das Ende eines Behandlungszyklus.

In einer bevorzugten Ausführungsform wird die Zeitmesseinrichtung automatisch nach der Einfüllphase gestartet, zum Auslösen eines Alarms nach Ablauf einer vorgegebenen Dwell- oder Verweilzeit der Dialyselösung im Peritoneum um den Patienten zu erinnern, eine erneute Drainagephase (16) und somit einen neuen Behandlungszyklus einzuleiten.

Um eine möglichst vollständige Leerung des Peritoneums zu erreichen, kann nach dem Erkennen des Drainage-Endes eine Anweisung an den Patienten erfolgen, welche ihn auffordert, aufzustehen und Schüttelbewegungen durchzuführen und/oder eine Wartezeit einzuhalten, so dass auch noch eventuell nachlaufendes Dialysat drainiert werden kann, bevor eine Dekonnektion erfolgt. Die Wartezeit kann in der Regel 1 bis 15 Minuten, vorzugsweise 5 bis 10 Minuten betragen.

In einer weiteren besonderen Ausführungsform kann über eine Zeitmesseinrichtung, zum Beispiel in Form einer Uhr mit Zeit- und Datumsanzeige, der Zeitpunkt, insbesondere das Datum und die Uhrzeit, der CAPD-Behandlung und/oder des Beutelwechsels erfasst werden.

Mittels der Zeitmesseinrichtung kann auch die aktuelle Wartezeit, vor der Dekonnektion, angezeigt werden.

Die Daten können intern auf einem Datenträger festgehalten werden oder auf einen Datenträger in einem externen Gerät übertragen werden. Durch den gespeicherten Zeitpunkt der letzten Behandlung und/oder des Beutelwechsels ist es möglich, den Patienten oder Anwender an den Beginn der nächsten Behandlung und/oder des nächsten Beutelwechsels zu erinnern. Die Erinnerung kann zum Beispiel durch ein optisches oder akustisches Signal, beispielsweise durch einen Alarm oder eine SMS oder ähnliche Hinweise, erfolgen, wodurch die Gefahr, dass ein Beutelwechsel vergessen wird, nahezu ausgeschlossen werden kann. Sollte ein Beutelwechsel trotz dessen nicht durchgeführt werden, so kann mittels des gespeicherten Zeitpunkts eine Alarmmeldung an den behandelnden Arzt oder das Pflegpersonal übermittelt werden.

Darüber hinaus ist es mittels der Zeitmesseinrichtung ebenfalls möglich, den Behandlungsverlauf und die Messdaten zur Dokumentation mit Uhrzeit und Datum auf einem Speichermedium, zum Beispiel für eine spätere Auswertung und Weiterverarbeitung der Daten, festzuhalten. In einer weiteren bevorzugten Ausführungsform kann die Sensorik (2) mit einer externen Datenerfassungs- und verarbeitungsvorrichtung kommunizieren, welche ebenfalls Daten von weiteren Geräten empfangen kann. Dabei kann es sich bei den weiteren Geräten zum Beispiel um eine Personenwaage, ein Blutdruckmessgerät oder einen Body Composition Monitor (BCM) handeln, die ebenfalls mit der externen Datenerfassungs- und verarbeitungsvorrichtung kommunizieren. Die externe Datenerfassungs- und verarbeitungsvorrichtung dient dabei als zentraler Daten-Hub, der wiederum auch Daten an die externen Geräte verschicken kann. Der zentrale Daten-Hub kann aber auch Bestandteil der Sensorik (2), insbesondere der Auswerteeinheit (9), sein. Durch die Anknüpfung von weiteren Geräten über einen zentralen Daten-Hub ist es möglich, eine Vielzahl von zusätzlichen Messdaten zu berücksichtigen, die Auskunft über den Behandlungsverlauf und das Therapieziel geben können.

### Legende:

- 1: gravimetrisch arbeitendes und manuell bedienbares CAPD-System
- 2: Sensorik
- 3: 1. Sensor
- 4: 2. Sensor
- 5: Dialysatbeutel
- 6: Drainagebeutel
- 7: Fluidsteuerungsvorrichtung
- 8: Schlauchsystem
- 9: Auswerteeinheit
- 10: Anzeigeeinheit
- 11: Speichermedium
- 12: unbeladener Zustand
- 13: Beladephase
- 14: Entladephase
- 15: 1. Zwischenphase
- 16: Drain- bzw. Drainagephase
- 17: 2. Zwischenphase
- 18: Spül- bzw. Primephase
- 19: 3. Zwischenphase
- 20: Einfüll- bzw. Fillphase
- 21: 4. Zwischenphase
- 22: 1. Streckenverlauf
- 23: 2. Streckenverlauf
- 24: Ausgangspunkt bzw. 0-Punkt
- 25: Haltevorrichtung

## Patentansprüche

1. Sensorik (2) an der ein gravimetrisch arbeitendes und manuell bedienbares CAPD-System (1) angebracht ist und mit der eine Veränderung gemessen werden kann, **dadurch gekennzeichnet, dass** die Sensorik zwei Gewichtssensoren (3, 4) umfasst und mit einer Auswerteeinheit (9) verbunden ist, wobei die Auswerteeinheit (9) derart angepasst ist um die mit den beiden Gewichtsensoren ermittelten Messdaten umzuwandeln um einen Phasenabschnitt und/oder Phasenübergang zu einem bestimmten Zeitpunkt zu bestimmen und zum Ablegen der Daten auf einem Speichermedium (11).

2. Sensorik (2) nach Anspruch 1, **dadurch gekennzeichnet, dass** die Messdaten zur Bestimmung der Veränderung eines Status einer Dialysebehandlung zu einem bestimmten Zeitpunkt, mittels der Auswerteeinheit (9) gegeneinander abgeglichen werden.

3. Sensorik (2) nach Anspruch 1, **dadurch gekennzeichnet, dass** die Messdaten der zwei Gewichtssensoren (3, 4) zeitgleich gegeneinander abgeglichen werden.

4. Sensorik (2) nach Anspruch 1, **dadurch gekennzeichnet, dass** die Messdaten der zwei Gewichtssensoren (3, 4), kontinuierlich gegeneinander abgeglichen werden.

5. Sensorik (2) nach Anspruch 1, **dadurch gekennzeichnet, dass** mittels der Auswerteeinheit (9) die Phasenabschnitte Belade-, Endlade- (13, 14), Drainage- (16), Spül- (18), und/oder Einfüllphase (20), die Phasenübergänge (15, 17, 19, 21) sowie die unbeladenen Zustände (12) bestimmt werden.

6. Sensorik (2) nach Anspruch 1, **dadurch gekennzeichnet, dass** mittels der Auswerteeinheit (9) die Phasenübergänge zwischen einer Belade-(13) und Drainagephase (16), einer Drainage- (16) und Spülphase (18), einer Spül- (18) und Einfüllphase (20) und/oder einer Einfüll- (20) und Entladephase (14) bestimmt werden.

7. Sensorik (2) nach Anspruch 1, **dadurch gekennzeichnet, dass** die Phasenabschnitte, Phasenübergänge und/oder Messdaten nummerisch, als Symbol oder als Textanzeige auf einer Anzeigeeinheit (10) ausgegeben werden.

8. Sensorik (2) nach Anspruch 1, **dadurch gekennzeichnet, dass** die, mittels der Sensoren (3, 4) ermittelten Messdaten, auf einem Speichermedium (11) festgehalten werden, wobei die Daten in einem internen Speichermedium (11) oder vorzugsweise auf einem austauschbaren Speichermedium (11) abgelegt werden oder besonders bevorzugt an ein externes Speichermedium (11) in einem separaten Datenverarbeitungsgerät übertragen werden.

9. Sensorik (2) nach Anspruch 8, **dadurch gekennzeichnet, dass** die auf einem Speichermedium (11) festgehaltenen Messdaten mittels kabelgebundener oder drahtloser Datenübertragung auf ein Speichermedium (11) übertragen werden.

10. Sensorik (2) nach einem der vorangegangenen Ansprüche, **dadurch gekennzeichnet, dass** die ermittelten und auf einem Speichermedium (11) festgehaltenen Messdaten mittels einer internen Auswerteeinheit (9) oder einer externen Datenverarbeitungsvorrichtung zur Fehlererkennung, zur Berechnung von einzelnen Behandlungsparametern und/oder durch den Abgleich mit Messdaten aus vorangegangenen Behandlungen, weiterverarbeitet werden.

11. Verfahren zur Messung einer Veränderung mittels einer Sensorik (2), die an ein gravimetrisch arbeitendes und manuell bedienbares CAPD-System (1) angebracht ist, **dadurch gekennzeichnet, dass** die Sensorik (2) zwei Gewichtssensoren (3, 4) umfasst und mit einer Auswerteeinheit (9) verbunden ist, wobei die Auswerteeinheit (9) die mit den Gewichtssensoren (3, 4) ermittelten Messdaten umwandelt um einen Phasenabschnitt und/oder Phasenübergang zu einem bestimmten Zeitpunkt zu bestimmen und die Daten auf einem Speichermedium (11) festgehalten werden.

12. Verfahren nach Anspruch 11, **dadurch gekennzeichnet, dass** die zwei Gewichtssensoren (3, 4) zu einem bestimmten Zeitpunkt Messdaten aufnehmen und diese gegeneinander abgeglichen werden.

13. Verfahren nach Anspruch 11, **dadurch gekennzeichnet, dass** die Messdaten von den zwei Gewichtssensoren (3, 4) zeitgleich zueinander aufgenommen und gegeneinander abgeglichen werden.

14. Verfahren nach Anspruch 11, **dadurch gekennzeichnet, dass** die Messdaten von den zwei Gewichtssensoren (3, 4) kontinuierlich aufgenommen und kontinuierlich gegeneinander abgeglichen werden.

15. Verfahren nach Anspruch 11, **dadurch gekennzeichnet, dass** sich aus den Messdaten der zwei Gewichtssensoren (3, 4) über die Zeit jeweils ein Streckenverlauf (22, 23) ergibt, wobei die beiden Streckenverläufe (22, 23) gegeneinander abgeglichen und in einer Auswerteeinheit (9) umgewandelt werden um für einen bestimmten Zeitpunkt einen Phasenabschnitt und/oder Phasenübergang auf einer Anzeigeeinheit (10) auszugeben.

16. Gravimetrisch arbeitendes und manuell bedienbares CAPD-System mit einer Sensorik zur Messung einer Veränderung nach Anspruch 1.

## Claims

1. A sensor system (2), on which a gravimetrically operating and manually operable CAPD system (1) is mounted and with which a change can be measured, **characterized in that** the sensor system comprises two weight sensors (3, 4) and is connected to an evaluation unit (9),
wherein the evaluation unit (9) is adjusted to convert the measured data determined by the two weight sensors in order to determine a phase segment and/or phase transition at a certain point in time and for storing the data on a memory medium (11).

2. The sensor system (2) according to claim 1, **characterized in that** the measured data are compared with one another to determine the change in status of a dialysis treatment at a certain point in time by means of the evaluation unit (9).

3. The sensor system (2) according to claim 1, **characterized in that** the measured data of the two weight sensors (3, 4) are compared with one another at the same time.

4. The sensor system (2) according to claim 1, **characterized in that** the measured data from the two weight sensors (3, 4) are compared continuously with one another.

5. The sensor system (2) according to claim 1, **characterized in that** the phase segments of loading, unloading (13, 14), draining (16), rinsing (18) and/or filling phase (20), the phase transitions (15, 17, 19, 21) as well as the unloaded conditions (12) are determined.

6. The sensor system (2) according to claim 1, **characterized in that** the phase transitions between a loading phase (13) and a draining phase (16), between a draining phase (16) and a rinsing phase (18), between a rinsing phase (18) and a filling phase (20) and/or between a filling phase (20) and an unloading phase (14) are determined by the evaluation unit (9).

7. The sensor system (2) according to claim 1, **characterized in that** the phase segments, phase transitions and/or measured data are output numerically, as symbols or as a text display on a display unit (10).

8. The sensor system (2) according to claim 1, **characterized in that** the measured data ascertained by means of the sensors (3, 4) are recorded on a memory medium (11), wherein the data are stored in an internal memory medium (11) or are preferably stored on a replaceable memory medium (11) or are preferably transferred to an external memory medium (11) in a separate data processing device in particular.

9. The sensor system (2) according to claim 8, **characterized in that** the measured data recorded on a memory medium (11) is transferred to a memory medium (11) by hardwired or wireless data transmission.

10. The sensor system (2) according to any one of the preceding claims, **characterized in that** the measured data determined and recorded on a memory medium (11) are processed further by means of an internal evaluation unit (9) or an external data processing device for error detection, for calculation of individual treatment parameters and/or by comparison with measured data from preceding treatments.

11. A method for measuring a change by means of a sensor system (2) mounted on a gravimetrically operating and manually operable CAPD system (1), **characterized in that** the sensor system (2) comprises two weight sensors (3, 4) and is connected to an evaluation unit (9), wherein the evaluation unit (9) converts the measured data detected by the weight sensors (3, 4) to determine a phase segment and/or a phase transition at a certain point in time, and the data are recorded on a memory medium (11).

12. The method according to claim 11, **characterized in that** the two weight sensors (3, 4) record measured data at a certain point in time and compare them with one another.

13. The method according to claim 11, **characterized in that** the measured data from the two weight sensors (3, 4) are recorded simultaneously and compared with one another.

14. The method according to claim 11, **characterized in that** the measured data are recorded continuously by the two weight sensors (3, 4) and are compared continuously with one another.

15. The method according to claim 11, **characterized in that** a curve (22, 23) is obtained from the measured data of the two weight sensors (3, 4) as a function of time, wherein the two curves (22, 23) are compared with one another and converted in an evaluation unit (9) to output a phase segment and/or a phase transition for a certain point in time to a display unit (10).

16. A gravimetrically operating and manually operable CAPD system having a sensor system for measuring a change according to claim 1.

## Revendications

1. Ensemble de capteurs (2) sur lequel est installé un système CAPD fonctionnant de manière gravimétrique et manoeuvrable manuellement (1) et grâce auquel une modification peut être mesurée, **caractérisé en ce que** l'ensemble de capteurs comprend deux capteurs de poids (3, 4) et est connecté à une unité d'exploitation (9),
l'unité d'exploitation (9) étant apte à convertir les données de mesure déterminées avec les deux capteurs de poids pour définir une section de phase et/ou une transition de phase à un moment donné et à enregistrer les données sur un moyen de mémoire (11).

2. Ensemble de capteurs (2) selon la revendication 1, **caractérisé en ce que**, pour déterminer la modification d'un état de traitement de dialyse à un moment donné, les capteurs sont alignés les uns par rapport aux autres au moyen de l'unité d'exploitation (9).

3. Ensemble de capteurs (2) selon la revendication 1, **caractérisé en ce que** les données de mesure des deux capteurs de poids (3, 4) sont alignées en même temps les unes par rapport aux autres.

4. Ensemble de capteurs (2) selon la revendication 1, **caractérisé en ce que** les données de mesure des deux capteurs de poids (3, 4) sont alignées en continu les unes par rapport aux autres.

5. Ensemble de capteurs (2) selon la revendication 1, **caractérisé en ce que**, au moyen de l'unité d'exploitation (9), les sections de phase suivantes : phase de chargement, déchargement (13,14), drainage (16), lavage (18), et/ou remplissage (20), les transitions de phase (15,17,19,21), de même que les états non chargés (12), sont définis.

6. Ensemble de capteurs (2) selon la revendication 1, **caractérisé en ce que**, au moyen de l'unité d'exploitation (9), les transitions de phase entre une phase de chargement (13) et une phase de drainage (16), une phase de drainage (16) et de lavage (18), une phase de lavage (18) et une phase de remplissage (20) et/ou une phase de remplissage (20) et une phase de déchargement (14) sont définies.

7. Ensemble de capteurs (2) selon la revendication 1, **caractérisé en ce que** les sections de phases, transitions de phases et/ou données de mesure sont éditées numériquement sous forme de symboles ou d'affichage texte sur une unité d'affichage (10).

8. Ensemble de capteurs (2) selon la revendication 1, **caractérisé en ce que** les données de mesure déterminées au moyen des capteurs (3, 4) sont consignées sur un moyen de mémoire (11), les données étant enregistrées dans un moyen de mémoire interne (11) ou de préférence dans un moyen de mémoire échangeable (11) ou, de manière particulièrement préférentielle, transférées à un moyen de mémoire externe (11) dans un appareil séparé de traitement de données.

9. Ensemble de capteurs (2) selon la revendication 8, **caractérisé en ce que** les données de mesure consignées sur un moyen de mémoire (11) sont transférées au moyen d'un transfert de données par câble ou sans fil vers un moyen de mémoire (11).

10. Ensemble de capteurs (2) selon une des revendications précédentes, **caractérisé en ce que** les données de mesure déterminées et consignées sur un moyen de mémoire (11) subissent un traitement ultérieur au moyen d'une unité d'exploitation interne (9) ou d'un dispositif externe de traitement des données pour la détection d'erreurs, pour le calcul de paramètres de traitement distincts et/ou par alignement avec des données de mesure issues de traitement précédents.

11. Procédé de mesure d'une modification au moyen d'un système de capteurs (2) qui est installé sur un système CAPD fonctionnant de manière gravimétrique et manoeuvrable manuellement (1), **caractérisé en ce que** le système de capteurs (2) comprend deux capteurs de poids (3, 4) et est connecté à une unité d'exploitation (9), l'unité d'exploitation (9) convertissant les données de mesure déterminées avec les deux capteurs de poids pour définir une section de phase et/ou une transition de phase à un moment donné et les données étant consignées sur un moyen de mémoire (11).

12. Procédé selon la revendication 11, **caractérisé en ce que** les deux capteurs de poids (3, 4) relèvent les données de mesure à un moment donné et que celles-ci sont alignées les unes par rapport aux autres.

13. Procédé selon la revendication 11, **caractérisé en ce que** les données de mesure des deux capteurs de poids (3, 4) sont relevées et alignées les unes par rapport aux autres simultanément.

14. Procédé selon la revendication 11, **caractérisé en ce que** les données de mesure des deux capteurs de poids (3, 4) sont relevées et alignées les unes par rapport aux autres en continu.

15. Procédé selon la revendication 11, **caractérisé en ce qu'**un itinéraire (22,23) résulte respectivement des données de mesure des deux capteurs de poids (3, 4) sur la durée, les deux itinéraires (22,23) étant alignés l'un par rapport à l'autre et convertis dans une unité d'exploitation (9) pour éditer pour un moment donné une section de phase et/ou une transition de phase sur une unité d'affichage (10).

16. Système CAPD fonctionnant de manière gravimétrique et manoeuvrable manuellement comprenant un système de capteurs pour la mesure d'une modification selon la revendication 1.
